# EUROPEAN PATENT APPLICATION

(11) **EP 1 800 599 A1**
(43) Date of publication of application: **27.06.2007**
(21) Application number: 05077971.9
(22) Date of filing: 23.12.2005
(51) Int. Cl.: A61B 5/0428, A61B 5/0408, A61B 5/00

(54) **Diagnostic electrode configuration**

(71) Applicant: Nederlandse Organisatie voor toegepast-natuurwetenschappelijk Onderzoek TNO, 2628 VK Delft (NL)
(72) Inventor: Nieuwkoop, Evert, 2641 MK Pijnacker (NL)
(74) Representative: Winckels, Johannes Hubertus F.

(57) **Abstract**

Sensor (1) for measuring electrical variables at the surface of a human or animal body, comprising three of more electrodes (2) in a geometrically regular arrangement, the sensor, moreover, comprising a support member (4), arranged to keep the electrodes together. The sensor may e.g. comprise a regular tripole (A, B, C) or quadrupole electrode configuration. The support member may have a rigid or a flexible constitution. The sensor may contain additional means (9) to detect orientation or position of the sensor w.r.t. the body, as well as means for energy scavenging.

## Description

### Field

The invention relates to a diagnostic electrode configuration e.g. as part of a system for making electrocardiograms etc.

### Background

Heart functions are often checked by means of an ECG (electrocardiogram) measurement. When doing this a couple of electrodes are attached at the limbs and the breast, which are connected with an ECG signal processing unit via wires. The wires to the electrodes are annoying because these regularly are pulled loose by the patient. Movements of and pulling forces at these wires cause a risk of wire break. Moreover, the patient is bound to the measurement location by all those wires. For that reason it will be preferred to incorporate the measuring electrodes in a wireless sensor network.

At measurements like ECG the electrodes are used to measure the potential difference between the electrodes. Wireless measuring a potential difference between not galvanically connected electrodes appears physically not to be realistic. In the (patent) literature developments can be observed to meet this problem. The first step is to continue with providing the electrodes with wires, which wires, however, are connected with a portable module that measures the potential differences and transfers those values via a transmitter to a receiver which is connected with the ECG data processing system. With such a "wireless" solution the mobility problem of patients is solved.

An extension to this is a system in which the wires of the breast electrodes are replaced by a sort of foil with electrodes that is attached upon the breast. Also in this system the electrodes are still connected galvanically with each other.

To evolve to a more wireless concept the use of dipole electrodes is introduced, at which the potential difference over short distance is measured and extrapolated to a potential difference over a longer distance, resulting in a conventional ECG image.

A disadvantage of the use of dipole electrodes is that not only placing at the right position is important but also the electrode's orientation w.r.t. the body.

### Summary

The invention proposes the use of sensors, each sensor comprising three or more electrodes in a geometrically regular arrangement., e.g. a regular tripole, quadrupole or (in general) multipole configuration. Such an electrode arrangement has a number of characteristics which make them very suitable for the present aim:
- The orientation of the sensor at the patient's body is no more important for an optimal signal amplitude. This is getting more and more important as in the future sportsmen, elderly people, patients etc. will more often place the electrodes their selves.
- The regular multipole electrode configuration offers the possibility to measure not only the amplitude but also the direction of the potential gradient w.r.t. the body, which offers enhanced possibilities for ECG research and the judgment of certain heart functions with one sensor device.
- The sensor will hardly be more expensive than a prior art sensor comprising a dipole electrode.

It is noted that the proposed new sensor is not restricted to ECG measurements but may also be applied in sensors for e.g. EEG (brain activity) or EMG (muscle activity) measurements.

Concluding, the present invention relates to a sensor for measuring electrical variables at the surface of a human or animal body, comprising three of more electrodes in a geometrically regular arrangement, the sensor, moreover, comprising a support member, arranged to keep the electrodes together. The geometrically regular arrangement may be a regular tripole or a regular quadrupole electrode configuration, offering the possibility to measure with one sensor both amplitude and direction of the potential gradient across the body. The support member may have a rigid or flexible constitution. The sensor primarily aims to be used in a (e.g. ECG, EEG, EMG) system for measuring electrical variables of a human or animal body, comprising one or more of such sensors.

### Exemplary Embodiment

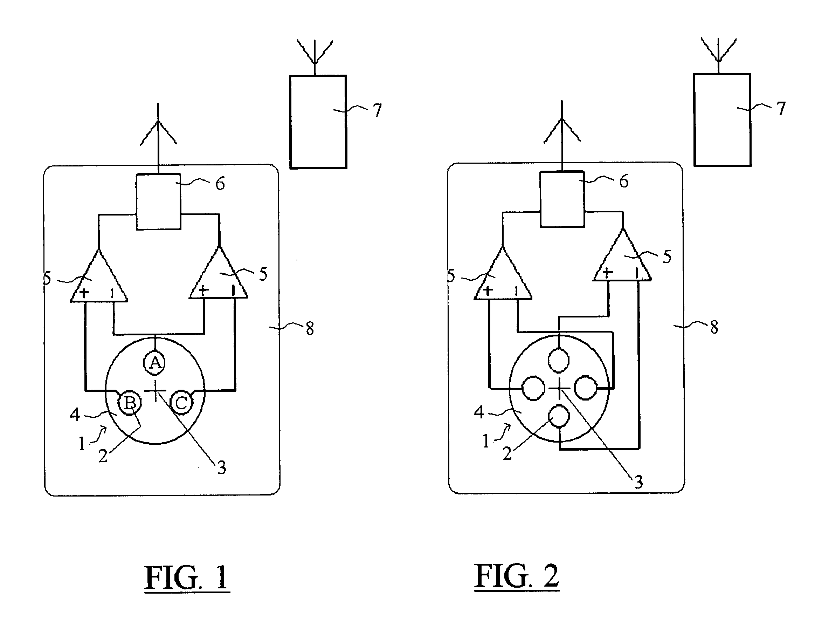

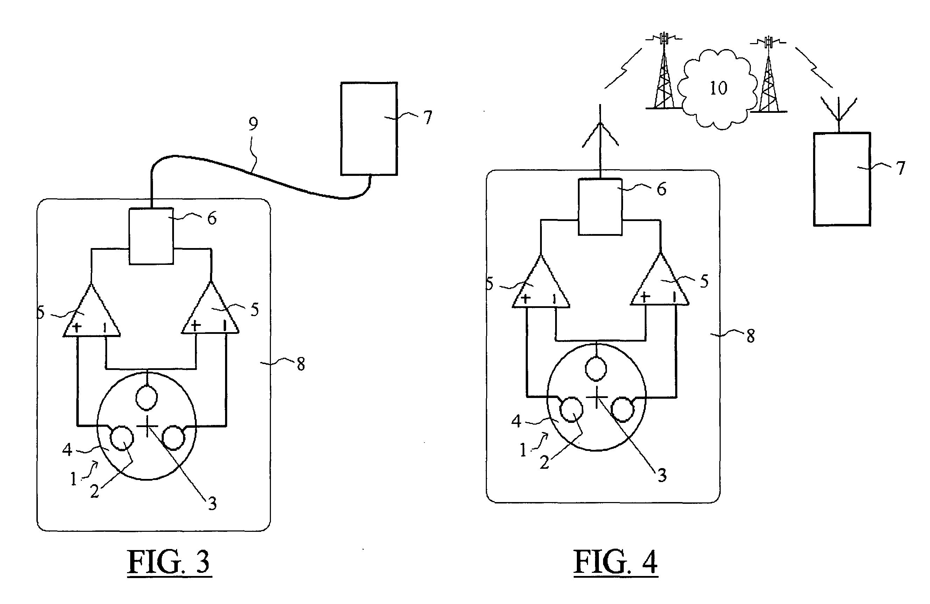

Figures 1, 2, 3, 4 and 5 show different embodiments of the invention.

Figure 1 shows a sensor 1 for measuring electrical variables at the surface of a human or animal body (not shown). The sensor 1 in figure 1 comprises a regular tripole electrode configuration, formed by three electrodes 2 - indicated by A, B, and C - in a geometrically regular arrangement around a centre 3. The sensor, moreover, comprises a support member 4, arranged to keep the electrodes 2 together and - at the same time - to isolate them electrically from each other. The support member 4 may have a rigid constitution or a flexible constitution, in both cases made suitable to be attached to the human or animal body under investigation.

The electrodes 2 are connected with two differential amplifiers 5, which are fit to measure the electrical potentials U_{A}-U_{B} (or U_{AB}) and U_{A}-U_{C} (or U_{AC}) at the body under investigation and to transfer them to a portable transmitter 6 which may be worn by the relevant person or animal. The transmitter 6 can also provide processing means, for example arranged to calculate from the measured potentials U_{A}-U_{B} (or U_{AB}) and U_{A}-U_{C} (or U_{AC}), the amplitude and direction of the potential gradient. Here the direction is the angle between the direction of maximum signal amplitude and a reference direction of the electrode configuration.
The transmitter 6 is capable to transmit the measured and/or calculated electrical signals to a receiver 7 which may be connected to further processing means (not shown). The human or animal body may be provided with one or more sensors 1, which have all their own transmitter 6, collecting all values measured by their respective sensor 1.

The electrodes, amplifiers and transmitter are assembled within one common housing 8, which may have the form of a small-sized "sensor button" that is attachable on the body which has to be monitored.

The sensor 1 in figure 2 comprises a regular quadrupole electrode configuration, formed by four electrodes 2 in a geometrically regular arrangement around a centre 3.

Transmission of the measuring values may be performed by means of a single wireless (radio, ZigBee ™, Bluetooth ™, InfraRed, inductive, etc.) path as illustrated in figures 1 and 2, by means of a wired connection 9 as shown in figure 3 (with the disadvantage of annoying wires for the patient which are prone to be pulled loose), or by means of a public or private (e.g. LAN) radio network 10.

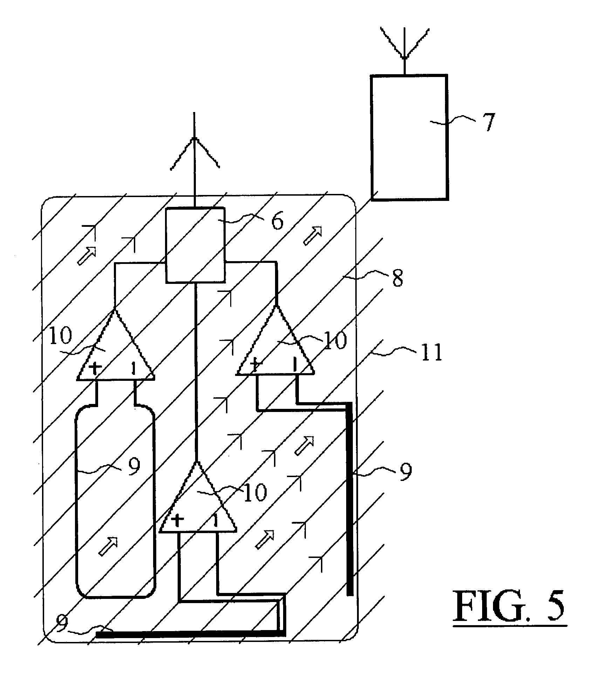

The amplifiers 5 and transmitter 6 may be powered by means of a battery which is enclosed in the housing 8 too or by means of some form of "energy scavenging", e.g. by conversion of thermal energy -based on temperature differences between the body under test and the environment- to electrical energy or by conversion of the energy from existing magnetic or electro-magnetic fields in the surrounding, e.g. from an alternating magnetic field 11 (figure 5) which may be provided for orientation/position detection purposes.

As illustrated in figure 5, the orientation and/or position of each "sensor button" 8 may be detected by means of e.g. one or more coils 9, in co-operation with means (not shown) which generate a alternating magnetic field 11 in a known direction with respect to the body (see arrows). The coils - one in plane x, one in plane y and one in plane z - are connected with amplifiers 10 and the detected field (strength, phase) of each coil 9 is transferred by transmitter 6 and receiver 7 to the processing means, which are adapted to compute, from the detected values, the orientation and position of the housing 8 of the sensor 1. By including position detecting means like coils 9, the processing means are capable to detect the exact orientation and position of the sensor electrodes 2 and thus in which direction -w.r.t. the body under investigation- the electrical signals have their maximum amplitude and thus to assess information about the electrical (heart) activity of said body.

## Claims

1. Sensor (1) for measuring electrical variables at the surface of a human or animal body, comprising three or more electrodes (2) in a geometrically regular arrangement, the sensor, moreover, comprising a support member (4), arranged to keep the electrodes together.

2. Sensor according to claim 1, the sensor comprising a regular tripole electrode (A, B, C) configuration.

3. Sensor according to claim 1, the sensor comprising a regular quadrupole electrode configuration.

4. Sensor according to claim 1, the support member having a rigid constitution.

5. Sensor according to claim 1, the support member having a flexible constitution.

6. System for measuring electrical variables of a human or animal body, comprising one or more sensors according to any of claims 1 - 5.

7. System according to claim 6, comprising position detection means (9,10) which are arranged to detect the orientation or position of the sensor electrodes (2) w.r.t. said human or animal body.

8. System according to claim 7, said position detection means comprising one or more coils (9), means which are fit to generate a magnetic field (11) in a known direction, the magnetic field detected by said one or more coils being transferred to processing means which are adapted to compute, from the detected magnetic field, the orientation or position of the sensor w.r.t. said human or animal body.

9. System according to claim 8, where said coils are also used for energy scavenging to obtain the required electrical power to operate the sensor system from said magnetic field (11).
